# EUROPEAN PATENT SPECIFICATION

(11) **EP 2 134 890 B1**
(45) Date of publication and mention of the grant of the patent: **07.10.2015**
(21) Application number: 08726328.1
(22) Date of filing: 03.03.2008
(51) Int. Cl.: A61B 17/88, A61F 2/06, A61F 2/07, D03D 25/00, D03D 3/02, D03D 15/00

(54) **FABRIC MEDICAL DEVICE HAVING A TAPERED TRANSITION AND METHOD OF MAKING**
MEDIZINTECHNISCHES FLÄCHENGEBILDE MIT SICH VERJÜNGENDEM ÜBERGANG UND HERSTELLUNGSVERFAHREN DAFÜR
DISPOSITIF POUR TISSU À USAGE MÉDICAL AVEC TRANSITION EFFILÉE ET PROCÉDÉ DE FABRICATION

(30) Priority: 02.03.2007 US 904631 P
(43) Date of publication of application: 23.12.2009
(73) Proprietor: Atex Technologies, Inc., Pinebluff NC 28373 (US)
(72) Inventor: SCHMITT, Peter, J., Franklin Square, New York 11010 (US); WEST, Anthony, Whispering Pines, North Carolina 28327 (US); GRECO, John, G., West End, North Carolina 27376 (US); NORRIS, Stephanie, B., Rocky Point, North Carolina 28457 (US)
(74) Representative: Hammler, Martin Franz
(86) International application number: PCT/US2008/002771
(87) International publication number: WO 2008/109019

(56) References cited:
- WO-A-01/89594
- WO-A-02/39928
- US-A- 3 991 249
- US-A- 6 136 022
- US-A1- 2003 078 650

## Description

### CROSS-REFERENCE TO RELATED APPLICATIONS

This application claims benefit of U.S. Provisional Patent App. No. 60/904,631, filed March 2, 2007.

### FIELD OF THE INVENTION

The present invention relates to a fabric medical device having a tapered transition and method of making the device. Embodiments of such a fabric medical device of the present invention may be advantageous for use as a bone filler material delivery device.

### BACKGROUND OF THE INVENTION

Tubular woven fabrics may be utilized in a variety of applications, including, for example, in devices useful in surgical procedures. One example of such a device is a tubular, woven, bone filler material delivery device. It may be desirable to provide such bone filler material delivery devices, or other tubular woven devices, in various shapes and sizes and such that the integrity of the fabric can be maintained during use of the device.

Conventional weaving techniques are commonly employed to fabricate tubular articles having various shapes. Tubular articles made utilizing conventional weaving techniques are generally made as single lengths or bifurcated structures. However, in conventional weaving processes, the transition from one diameter to another diameter can occur at a single point in the weave, creating a sudden change in the weaving pattern of the fabric in transition areas. Such sudden changes can create voids and gaps in the tubular article, which may be undesirable when the tubular article is to be used for containing and/or delivering a fluid or semi-fluid material. As a result, such weaving techniques have specific limitations as to the final shape of the article. That is, complex shapes, such as tubular "S-shaped" or frustoconical-shaped woven sections have not been attempted using conventional weaving techniques due to the impractibility of using those techniques for such articles, intensive labor, and resulting high cost to the consumer.

Examples of tubular woven articles include those disclosed in International Publication No. WO 02/39928 of Schmitt; U.S. Patent No. 3,991,249 of Yamashita et al.; U.S. Patent No. 6,136,022 of Nunez et al.; and U.S. Patent App. Publication No. US 2003/0078650 of Nunez et al.

For example, International Publication No. WO 02/39928 of Schmitt discloses single tubular woven or bifurcated prostheses having varying diameters and tapered transitions. The prostheses comprise a seam along the tapered edges, thereby providing a substantially fluid-tight transition between sections or extents of the prostheses. The seam may be located at an edge where fabric of the prosthesis tapers from one diameter to a different diameter and/or a point where the prosthesis splits such as a bifurcation. The seamed crotch may be used for tapered and non-tapered bifurcated grafts. The seam may be woven directly on a weaving loom or joined together after weaving is completed.

U.S. Patent No. 3,991,249 of Yamashita et al. discloses an improved fabric material for producing woven air bags utilized for protecting occupants in vehicles. The fabric material comprises a plurality of large tubular weave portions formed in the direction of the warp yarns and a stitched portion formed between two adjacent tubular weave portions. The stitched portion comprises at least one small tubular weave portion in which the warp yarns for constructing a face fabric of the tubular weave portion and the warp yarns for constructing a back fabric of the tubular weave portion are respectively used as the warp yarns for constructing a back fabric of a successive small tubular weave portion and as the warp yarns for constructing a face fabric of the successive small tubular weave portion in the stitched portion. The crossing angle of each warp yarn at a boundary portion between the large tubular weave portion and the stitched portion is smaller than the crossing angle of each weft yarn. This construction of the fabric material allows the space occupied by the stitched weave portion to be limited to a very small area, thereby increasing the bursting strength of the air bag. At the boundary portion between the large tubular weave portion and the stitched portion, the crossing angle of each warp yarn is smaller than the crossing angle of each weft yarn. As a result, the breaking, or tensile, strength of the fabric material along the warp yarn direction is stronger than that of the fabric material in the weft yarn direction in the tubular portion of the woven air bag. As a consequence of the smaller warp yarn crossing angles, when a tensile force is applied to the fabric, a larger shearing force is imparted to the weft yarn in comparison with the warp yarn and, therefore, if the same yarn is used for the warp yarn and the weft yarn and their densities are identical, the breaking strength of the fabric in the warp yarn direction is larger than that in the weft yarn direction. Thus, it is understood that the increase of the bursting strength of the air bag was mainly due to an increase in the density of warp yarns which increased the fabric strength in the warp yarn direction.

U.S. Patent No. 6,136,022 and U.S. Patent App. Publication No. US 2003/0078650 of Nunez et al. disclose continuously flat-woven implantable tubular prostheses have seamless woven sections which gradually change the number of warp yarns to smoothly transition, i.e., taper, from one diameter to another. Multi-diameter endoluminal grafts having a variety of shapes and configurations can be made using a seamless weaving process without unacceptable voids or gaps in the tubular wall. As a consequence, custom-made woven tubular articles can suffer the disadvantages of gaps created at transition points between portions of the article due to separation or splitting of warp yarns in those areas.

Some surgical procedures are increasingly becoming minimally invasive procedures in which the surgical site is accessed via a narrow incision or a percutaneous puncture to the site. In surgical procedures generally, and in minimally invasive procedures in particular, it may be desirable to utilize a device for delivering fluid or semi-fluid material to the surgical site that is proportioned so as to fit through a small opening. In such situations, the delivery device may have a decreasing diameter from the proximal portion of the device outside the patient's body to the distal portion of the device at the surgical site. In addition, devices such as a bone filler material delivery device may be angled to some degree so as to reach a target structure not directly in line with the surgical access pathway. In other procedures, it may be desirable to utilize a tubular woven device having an angle along the length of the device so as to keep the user's hands and/or the device out of the field of an imaging device, such as a fluoroscope, that may be used to monitor the procedure.

Because of the high viscosity of materials, such as bone cement, high pressures may be required to inject such materials from the delivery device into hard bony tissue at a target site. Such high pressures can cause the user to apply substantial force to the delivery device, creating a risk for rupturing the fabric of the tubular woven device.

One conventional bone filler material delivery device employs a gun-type injector in order to use the mechanical advantage of a lever to provide pressures high enough to deliver the material from a chamber of the device into a patient. In a vertebroplasty procedure, for example, bone filler material can be injected at high pressure, such as about 700 psi, into the interior of a vertebral body, without the prior formation of a cavity. Because high pressure is used, there is little opportunity to quickly and accurately adjust the flow of the bone filler material in reaction to bone volume and density conditions encountered. Momentum generated by high pressure-induced flow of bone filler material can continue to propel the material into the targeted bone site even after termination of the high pressure. That is, once the pressure-generating mechanism is triggered, conventional bone cement injection devices do not permit the injection volume or injection rate to be adjusted or controlled in real time. In addition, a lever-type injector can have the disadvantage of requiring heavy, complex mechanical components to achieve sufficiently high pressures for delivery of a viscous material.

Other conventional bone filler material delivery devices utilize a pneumatic means for injecting bone filler material into a patient. Pneumatic injector devices often provide inadequate control of the delivery of the bone filler material, and may pose safety concerns in applications such as vertebroplasty in which control of the injected material is critical. As a result of the relatively high pressure that conventional procedures rely upon, coupled with the effective lack of a short response time, the targeted bone interior can suddenly overfill. Excess filling material can be forced outside the bone interior, and into adjoining tissue regions, where the presence of filler material is not desired.

Thus, it may be desirable for a tubular woven article, such as a bone filler material delivery device, to comprise transitions between portions having varying diameters and/or branching segments that avoid gaps and voids in the tubular wall of the device, and thus have an improved barrier against leakage in those transition areas and/or branching segments. It may be further desirable for such a tubular woven bone filler material delivery device to allow greater control of the rate of injecting a bone filler material.

### SUMMARY OF THE INVENTION

Some embodiments of a fabric medical device having a tapered transition and/or method of making the device of the present invention can include a plurality of warp yarns and fill yarns woven together to form a first tubular extent having a first diameter and a formed shape, a second tubular extent having a second, different diameter and a formed shape, and a transition tubular extent having a graduated diameter between the first and second tubular extents. A tapered edge can be formed along the transition tubular extent by a weaving pattern in which a graduated number of warp yarns are disengaged along the transition tubular extent. A seam can be woven along the tapered edge that is configured to provide a substantially fluid-tight transition between the first tubular extent and the second tubular extent. Such an embodiment of a device can further include the warp yarns in at least the transition tubular extent having a tenacity higher than the tenacity of the fill yarns.

In some embodiments, the warp yarns can include top layer warp yarns and bottom layer warp yarns, and the seam can comprise the top layer warp yarns and the bottom layer warp yarns woven together along the tapered edge. In some embodiments, the warp yarns can be disengaged at a sufficiently high ratio relative to the fill yarns such that the diameter of the transition tubular extent graduates at an angle of at least 45 degrees between the first and second tubular extents. In some embodiments of a fabric medical device according to the present invention, the device can be a bone filler delivery device adapted to deliver bone filler to a bony area in an internal body region.

### BRIEF DESCRIPTION OF THE DRAWINGS

FIG. 1 is a weaving schematic showing a plain tubular weave pattern in an embodiment of the present invention.
FIG. 2 is a weaving schematic showing a weaving pattern used in producing a tapered edge in a seamless tubular article.
FIG. 3 is a weaving schematic showing a woven seam weave pattern utilized to produce a tapered edge in a tubular article in an embodiment of the present invention.
FIG. 4 is a weaving schematic showing an open edge weave pattern utilized to produce an open edge in a tubular article in an embodiment of the present invention.
FIG. 5 is a weaving schematic showing an open crotch weave pattern utilized to produce a tapered edge at a split in a tubular article in an embodiment of the present invention.
FIG. 6 is a weaving schematic showing a stitched crotch weave pattern utilized to produce a stitched crotch in a tubular article in an embodiment of the present invention.
FIG. 7 is a view of a frustoconical-shaped tubular article in an embodiment of the present invention.
FIG. 8 is a view of a tubular article having a first tubular extent having a first constant diameter, a transition tubular extent having a graduated diameter, and a second tubular extent having a second constant diameter in an embodiment of the present invention.
FIG. 9 is a view of a tubular article having a first, elongated tubular extent having a first constant diameter continuously woven to a transition tubular extent having a graduated diameter in the form of an inward taper in an embodiment of the present invention.
FIG. 10 is a view of a tubular article having a transition tubular extent having a graduated diameter in the form of an outward flare continuously woven to an elongated tubular extent having a constant diameter in an embodiment of the present invention.
FIG. 11 is a view of a sinusoid-shaped tubular article in an embodiment of the present invention.
FIG. 12 is a view of a tubular article having an angular transition between a first tubular extent having a first constant diameter and a transition tubular extent, and an angular transition between the transition tubular extent and a second tubular extent having a second constant diameter in an embodiment of the present invention.
FIG. 13 is a view of a bifurcated tubular article having secondary tubular extents having the same diameter in an embodiment of the present invention.
FIG. 14 is a view of bifurcated tubular article having secondary tubular extents having different diameters in an embodiment of the present invention.
FIG. 15 is a view of a bone filler material delivery device having a continuously woven transitional tubular extent in an embodiment of the present invention.
FIG. 16 is a view of a bone filler material delivery device having a continuously woven transitional tubular extent in an embodiment of the present invention.
FIG. 17 is a close-up perspective view of the embodiment of the bone filler material delivery device having a continuously woven transitional tubular extent shown in Fig. 17.

### DETAILED DESCRIPTION OF THE INVENTION

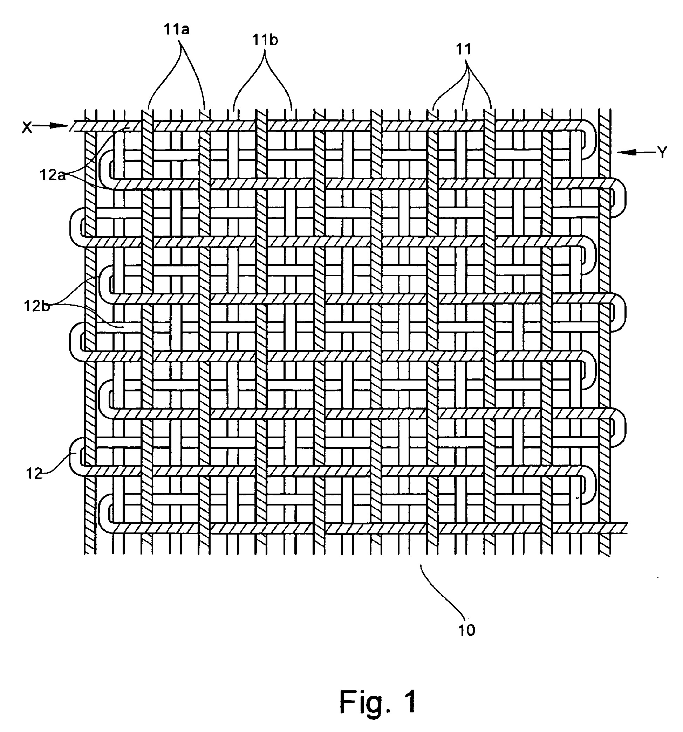

Some embodiments of the present invention can provide a fabric medical device having a tapered transition and/or method of making the device. Illustrative embodiments of a fabric medical device having a tapered transition and/or method of making the device of the present invention are shown in Figs. 1-17.

As used in this specification and the appended claims, the singular forms "a," "an," and "the" include plural referents unless the context clearly dictates otherwise. Thus, for example, the term "a device" is intended to mean a single device or a plurality of devices, and "a seam" is intended to mean a single seam or a plurality of seams.

Some embodiments of the present invention can comprise a tubular article having a tapered area, or transition area, that tapers from a larger diameter to a smaller diameter. In certain embodiments, the tubular article can comprise a woven fabric. The tubular article can be, for example, a bone filler delivery device useful for delivering bone filler material, such as a bone cement, to a bony area in an internal region of a human or animal. An embodiment of a bone filler delivery device having a tapered transition may be particularly useful for delivering bone filler material to a treatment site in minimally invasive surgical procedures.

The tapered transitions can include a seam, or closure, along a tapered edge. In some embodiments, the seam can provide a substantially fluid-tight transition between a first tubular portion, or extent, and a second tubular portion, or extent. The seam may be located at an edge where fabric of the tubular article tapers from one diameter to a different diameter and/or at a point where the tubular article splits, such as with a bifurcation. For purposes herein, an edge is defined as an outer limit of the width of the tubular article along its longitudinal axis as the article is flat-woven on a loom. In certain embodiments, the seam can be woven directly on a loom by weaving together the top and bottom fabric portions of the tubular article. In other embodiments, the open edges of the transition portion can be joined together by various means after weaving is completed in order to form a seam, or closure. Embodiments of a tubular article of the present invention having such a seam, or closure, can provide the advantage of minimizing voids and gaps found along the tapered edges and at bifurcation point(s) of conventional tubular articles.

In some embodiments, the tubular article can be a tubular bone filler delivery device. The bone filler delivery device can include a first extent having a first constant diameter, a tapered transition extent continuously woven from the first extent, and a second extent having a second constant diameter smaller than the first constant diameter and that is woven continuously from the transition extent. In one illustrative embodiment, the device can have a length of approximately four to six inches (10.2 cm to 15.2 cm). In such an embodiment, the length of each extent can vary. That is, in various embodiments, each extent can be longer, shorter, or the same length as the other extents. For example, the first constant diameter extent may be two inches (5.1 cm) in length, the tapered transition extent two inches (5.1 cm) in length, and the second constant diameter extent two inches (5.1 cm) in length. Alternatively, the first constant diameter extent may be one inch (2.54 cm) in length, the tapered transition extent four inches (10.2 cm) in length, and the second constant diameter extent one inch (2.54 cm) in length. Each extent in embodiments of a tubular article can be any length desired for the end use of the article.

Some embodiments of a tubular article of the present invention can include various degrees, or angles, of taper in transition areas. In an illustrative embodiment, a tubular bone filler delivery device comprising woven fabric can taper gradually from one end of the device to the opposite end of the device. At one, larger end of the tube, the device can have a diameter of, for example, about 25-30 mm. The device can include a taper in which the fabric is woven so as to gradually reduce the diameter of the tube along its length. The opposite, smaller end of the device can have a diameter of, for example, about 4-8 mm. A bone filler delivery device having such dimensions may be useful for positioning the device through a surgical access path in a minimally invasive procedure.

In some embodiments, the tubular bone filler delivery device can include a tapered transition area in which the degree, or angle, of transition changes abruptly. For example, the tapered transition area can include an angle of transition of about 45-135 degrees. In one such embodiment, the tubular bone filler delivery device can include a first extent having a first constant diameter, a tapered transition extent continuously woven at about a 90 degree angle from the first extent, and a second extent having a second constant diameter smaller than the first constant diameter and that is woven continuously from the transition extent. Embodiments of the present invention can advantageously provide tubular woven, single-lumen or bifurcated articles that can be produced with varying diameters and tapered transitions. As a result, the present invention can advantageously provide embodiments of tubular articles in complex shapes.

In weaving processes useful in making embodiments of tubular articles or the present invention, yarns can be interwoven in different directions to create the tubular fabric. For example, a set of warp yarns can run lengthwise parallel to the selvages, or edge portions, and represent the width of the product being woven. Fill yarns run from selvage to selvage at right angles to the warp and are interlaced between the warp yarns. The fill yarn can be woven along the length of the warp yarns, with each successive pass of the fill yarn across the warp yarns for each side of the tube representing one machine pick. Weaving one fill yarn along the entire circumference of the tube, for example, one filling pick, requires two picks of the weaving machine. Thus, two machine picks represent one filling pick in a tubular woven structure. As such, the fill yarn can be woven along the length of the warp yarns for a multiple number of machine picks. The resulting woven product is defined in length by the number of filling picks of the fill yarn and defined in width by the number of warp yarns in which the fill yarn is woven between.

In embodiments of the present invention, warp ends, or yarns, and pick ends, or fill yarns, can be uniform throughout the entire tubular article, including in the tapered transitions areas. That is, the density of yarns can be uniform throughout each tubular extent in the tubular article, including, for example, a first constant diameter tubular extent, a second constant diameter tubular extent, and a tapered transition tubular extent between the first and second tubular extents. Such embodiments of a tubular article having tapered seam transitions can have more uniform characteristics such as porosity, strength, flexibility, and thickness along the length of the tubular article as compared to conventional tubular articles. As a result, the tapered transition areas, such as a seamed transition, can be essentially fluid-tight, or non-permeable. Thus, embodiments of a tubular article having such seams at transition areas, such as tapers and furcations, can provide increased strength to the tubular article and an improved barrier against leakage in those transition areas.

In various embodiments, a tubular article of the present invention can include one or more tapered transition areas adapted to provide a reduction in diameter between the ends of the device to facilitate inserting the device through a percutaneous pathway to an internal body region and to deliver a fluid or semi-fluid material to the internal body region. One such embodiment can comprise a bone filler delivery device. An embodiment of a bone filler delivery device of the present invention can be utilized, for example, in vertebroplasty and/or kyphoplasty procedures.

Vertebroplasty is a minimally invasive surgical procedure for reducing a vertebral fracture. In a vertebroplasty procedure, or other vertebral body repair procedure, a device can be percutaneously inserted into cancellous bone inside a vertebral body. The device can be used to create a void, or cavity, in the cancellous bone. Once the void is created, bone filler material can be delivered into the cavity to provide structural support to the cortical bone of the vertebral body. Kyphoplasty is a minimally invasive surgical procedure for reducing a vertebral fracture similar to a vertebroplasty. In addition to reducing a vertebral fracture, kyphoplasty procedures can include restoring height to an injured or diseased vertebra.

Some embodiments of a tubular article having a tapered transition area can be utilized in applications other than as a bone filler delivery device. Some embodiments of a tubular article having a tapered seam transition may be adapted for use in a variety of clinical applications, for example, in cardiovascular, gastrointestinal, genitourinary, gynecologic, hepatobiliary, endocrine, otolaryngologic, pulmonary, and other intra- and inter-organ regions. Such tubular articles may be curved, tapered, or otherwise adapted for use in different anatomic regions. For example, certain embodiments may be utilized to instill other fluids and/or semi-fluid material into various internal body regions. One embodiment may be used to irrigate and/or to instill an antibiotic into an internal body region. Another embodiment may be used to inject a gel material into an implanted breast implant. Yet another embodiment may be used to transfuse blood products into a person's cardiovascular system. Some embodiments of a tubular article having a tapered transition area can be utilized for topical application of a material. Other embodiments can be utilized in other clinical applications and/or as well as in settings apart from clinical environments and uses.

Embodiments of a tubular article having a tapered transition area can comprise various materials. For example, a tubular article such as a bone filler delivery device may comprise an acrylic material and/or other synthetic and/or natural fibers. In certain embodiments, the tubular article can be disposable.

In one illustrative embodiment, a woven tubular article can comprise a plurality of warp yarns and fill yarns, a first tubular extent having a first diameter, a second tubular extent having a second diameter different from the first diameter, and a transition tubular extent between the first and second tubular extents. The transition tubular extent can have a tapered edge along the transition extent formed by a weaving pattern in which a graduated number of warp yarns are disengaged as weaving proceeds. This weaving pattern can thus provide a graduated change in the number of warp yarns to form a graduated diameter along the transition tubular extent between the first and second tubular extents. A seam can be woven along the tapered edge, such that the seam provides a substantially fluid-tight transition between the first tubular extent and the second tubular extent. Such an embodiment can be, for example, a bone filler delivery device. In certain embodiments, the tubular article can be flat-woven.

In some embodiments, the first, second, and transition tubular extents can be woven together, and the seam can be woven during weaving of the transition tubular extent. In certain embodiments, the plurality of warp yarns can further comprise top layer warp yarns and bottom layer warp yarns. The seam can comprise the top layer warp yarns and the bottom layer warp yarns woven together along the tapered edge.

The diameter of the first tubular extent and the diameter of the second tubular extent can each be defined by a different number of warp yarns. The first tubular extent can comprise a constant first diameter, and the second tubular extent can comprise a constant second diameter different than the first diameter. In some embodiments, the ratio of disengaged warp yarns to fill yarns can allow the tapered edge to have an angle greater than 45 degrees relative to the first and/or second tubular extents. For example, the ratio of disengaged warp yarns to fill yarns can allow the tapered edge to have an approximately 90 degree angle relative to the first and/or second tubular extents.

Embodiments of the tubular article can comprise various shapes. For example, embodiments of the tubular article can have a frustoconical shape, in which the first tubular extent diameter is graduated, the second tubular extent diameter is graduated, the transition tubular extent diameter is graduated, and the diameters of the first, second, and transition tubular extents are each graduated in the same direction. In another embodiment, the tubular article can have an "S" shape, in which the tapered edge comprises two opposing edges. The transition tubular extent can have a curve in one direction in which the warp yarns at a first opposing edge are successively disengaged, and corresponding warp yarns at a second opposing edge are successively engaged. The "S"-shaped transition tubular extent can have a curve in the opposite direction in which the warp yarns at the first opposing edge are successively engaged, and corresponding warp yarns at the second opposing edge are successively disengaged. In another embodiment, the tubular article can include an inward taper, in which the first tubular extent diameter is constant and the second tubular extent comprises the transition tubular extent. In still another embodiment, the tubular article can include an outward flare, in which the first tubular extent comprises the transition tubular extent and the second tubular extent diameter is constant.

In some embodiments, the woven tubular article can include two secondary tubular extents, each having a diameter different than the first tubular extent first diameter. The transition tubular extent can extend between the first tubular extent and the two secondary tubular extents, and have a tapered edge and a graduated diameter. The two secondary tubular extents can bifurcate from the transition tubular extent such that the tapered edge forms a crotch in the transition tubular extent between the two secondary tubular extents. A substantially fluid-tight seam can be woven along the crotch in the transition tubular extent. The seam can comprise the top layer warp yarns and the bottom layer warp yarns woven together along the crotch. Each of the two secondary tubular extents can have the same diameter or different diameters.

In another embodiment, the woven tubular article can comprise a plurality of warp yarns and fill yarns, a first tubular extent having a first diameter, and a second tubular extent having a second diameter different from the first diameter, such that the first and second tubular extents are spaced apart to define an open transition extent therebetween. The open transition extent can have an unwoven tapered edge along the transition extent formed by a weaving pattern having a graduated change in the number of warp yarns. The graduated change in the number of warp yarns can comprise a graduated disengagement of predetermined warp yarns from the weaving pattern.

In an embodiment having an open transition extent, the fill yarns can comprise top layer fill yarns and bottom layer fill yarns, and the first, second, and transition tubular extents can be woven together such that the fill yarns at the tapered edge are woven onto the same layer, leaving the transition tubular extent open.

The unwoven, open tapered edge can then be closed with a seam along the tapered edge. The seam can comprise a seam forming means to close the open transition extent into a tube after weaving is completed. In various embodiments, the seam forming means can comprise stitching, or sewing, gluing, stapling, welding, and/or the like along the tapered edge of the open transition extent. The seam can thus provide a substantially fluid-tight transition between the first tubular extent and the second tubular extent.

The tubular article having an unwoven tapered edge along the open transition extent that is closed with a fluid-tight seam after weaving is completed can have various shapes. For example, such a tubular article can have a frustoconical shape or an "S" shape. In some embodiments, the transition tubular extent comprises an inward taper. In other embodiments, the transition tubular extent can comprise an outward flare.

In another embodiment, the woven tubular article can comprise a plurality of warp yarns and fill yarns, a first tubular extent having a first diameter, a plurality of secondary tubular extents, each woven at a transition with the first tubular extent, and an open crotch formed at the transition between the first tubular extent and the plurality of secondary tubular extents. The open crotch can be formed by a weaving pattern that disengages a graduated number of predetermined warp yarns along the transition between the first tubular extent and plurality of secondary tubular extents. A seam can be formed along the crotch such that the seam provides the transition between the first tubular extent and the plurality of secondary tubular extents with a substantially fluid-tight closure.

In such an embodiment, the seam can comprise a seam forming means to close the open crotch after weaving is completed. In some embodiments, the seam forming means can comprise stitching sewn along the open crotch. In other embodiments, the seam forming means can comprise gluing, stapling, welding, and/or the like.

In certain embodiments, the plurality of secondary tubular extents can comprise a pair of secondary tubular extents defining a bifurcated structure. Each of the pair of secondary tubular extents can have the same diameter or a different diameter.

Referring to the Figures, FIG. 1 shows a plain tubular weave pattern 10 useful in making embodiments of tubular articles of the present invention. Warp yarns 11 are further shown as 11a indicating they are in the top layer of the weave and 11b indicating their presence in the bottom layer of the weave. Top layer warp yarns 11a and bottom layer warp yarns 11b run in a lengthwise direction in the tubular article and define the width of the article. Fill yarns 12 are further shown as top fill yarns 12a and bottom fill yarns 12b. These fill yarns are woven with the top and bottom warp yarns 11a and 11b as shown in FIG. 1. For example, a filling yarn shuttle (not shown) passes across warp yarns 11 while selected warp yarns 11 are lifted according to a specific weave pattern. In electronic weaving machines, such weave patterns can be programmed into the machine using software. In a plain tubular weave as depicted in FIG. 1, the shuttle first weaves top fill yarn 12a by passing across warp yarns 11 while certain warp yarns 11 are lifted. During travel of top fill yarns 12a, in direction X, for weaving of the top tubular body portion, the bottom warp yarns 11b are not lifted so as to prevent top fill yarns 12a from interweaving with bottom warp yarns 11b. Likewise, during passage of bottom fill yarns 12b, in direction Y, for weaving of the bottom tubular body portion, the top warp yarns 11a are always lifted such that bottom fill yarns 12b are not interwoven with top warp yarns 11a. Such a plain tubular weave pattern can be used to form single portions of embodiments of tubular articles that have a constant diameter. This weave pattern can be modified by gradually engaging or disengaging warp yarns to create tapers and/or shapes.

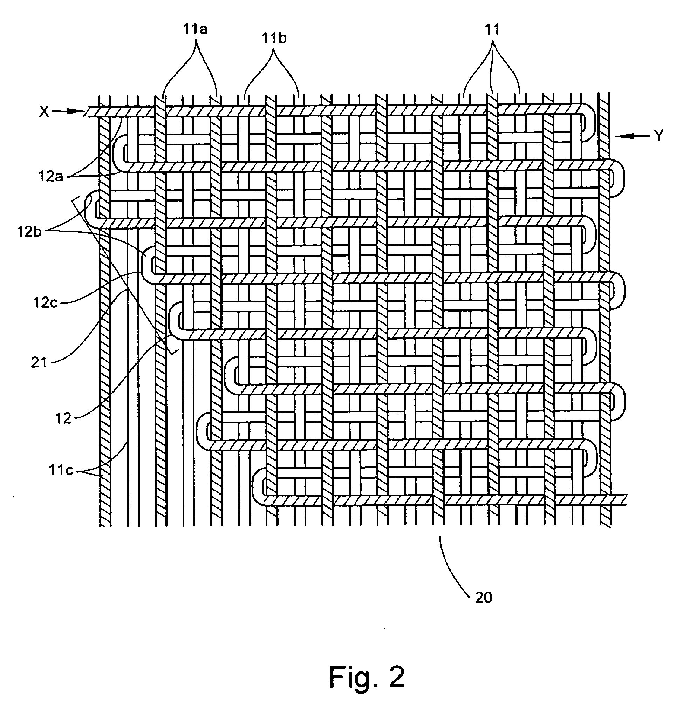

FIG. 2 is a weaving schematic depicting a weaving pattern 20 useful in making an embodiment of a seamless tubular article according to the present invention. As shown in FIG. 2, the tapered edge 21 can be formed by gradually disengaging the warp yarns 11a and 11b. Disengaging the warp yarns 11a and 11b can be accomplished by dropping the desired warp yarns, for example warp yarns 11c, such that the fill yarns 12 are not interwoven across the warp yarns 11 for that section of the pattern. This technique can produce a tapered edge 21 in a tubular article. This type of dropping of warp yarns in a gradual manner forms the transitional portion of the article. In continuous flat-weaving processes, the warp yarns can then be re-engaged during the weave pattern once the transitional section has been completed.

As described, in some embodiments of the present invention, transition from one diameter to another diameter can be accomplished by engaging and/or disengaging predetermined warp yarns from the weave pattern. Such disengaging or engaging of warp yarns can be gradual. However, such a transition can potentially be accomplished using any combination of numbers of warp yarns and fill yarns. Embodiments of tubular articles of the present invention allow varied rates of transition such that acute angles resulting from abrupt changes in the weaving pattern can be accommodated.

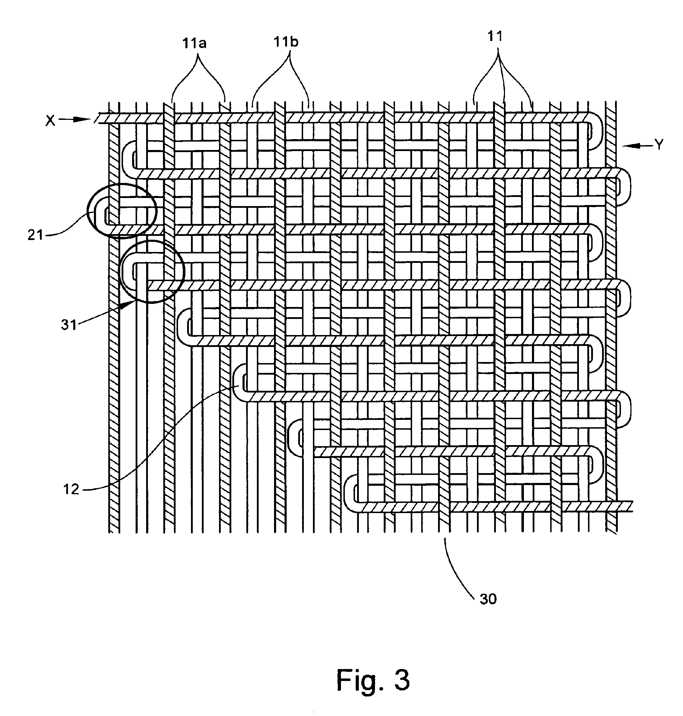

FIG. 3 shows a seam weave pattern 30 utilized in an embodiment of a method of the present invention to produce the edge 21 of a tubular article in an embodiment of the present invention. As shown in FIG. 3, the tapered tubular edge 21 can be formed by interweaving top layer warp yarns 11a and bottom layer warp yarns 11b together to form a seam, or "selvage," comprising a single layer fabric at the tapered edge 21. A minimum of one warp yarn from each of the top and bottom layers can be utilized to form the woven seam 31. Additional warp yarns from either or both layers may be utilized to increase the width of the seam 31. The greater the number of warp yarns utilized, the greater the size, or width, of the seam. In embodiments of the present invention, the size of the seam may be varied depending on the intended end use of the tubular article. In embodiments utilizing a woven seam, as shown in FIG. 3, the seam can be made while the tubular article is still on the weaving loom.

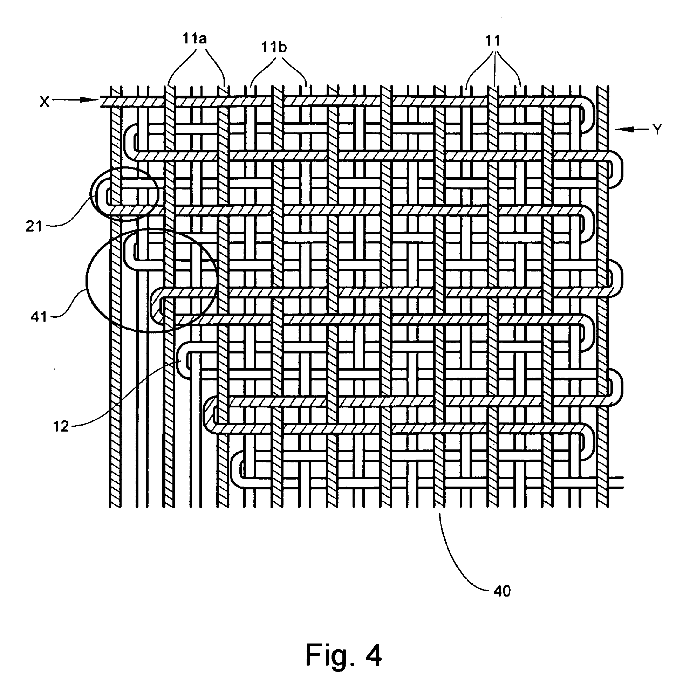

FIG. 4 shows an open edge weave pattern 40 utilized in an embodiment of a method of the present invention to produce an open edge 41 in a tubular article. As shown in FIG. 4, the open tapered edge 41 can be formed by causing the fill yarns 12 to remain on the same layer, either top or bottom, at the tapered tubular edge 21. In embodiments utilizing an open edge weave pattern, as shown in FIG. 4, a seam can generally be made after weaving is complete and the article is removed from the loom. The tapered edges may be sealed by sewing, welding, bonding, gluing, stapling, and/or other techniques suitable for sealing tubular articles.

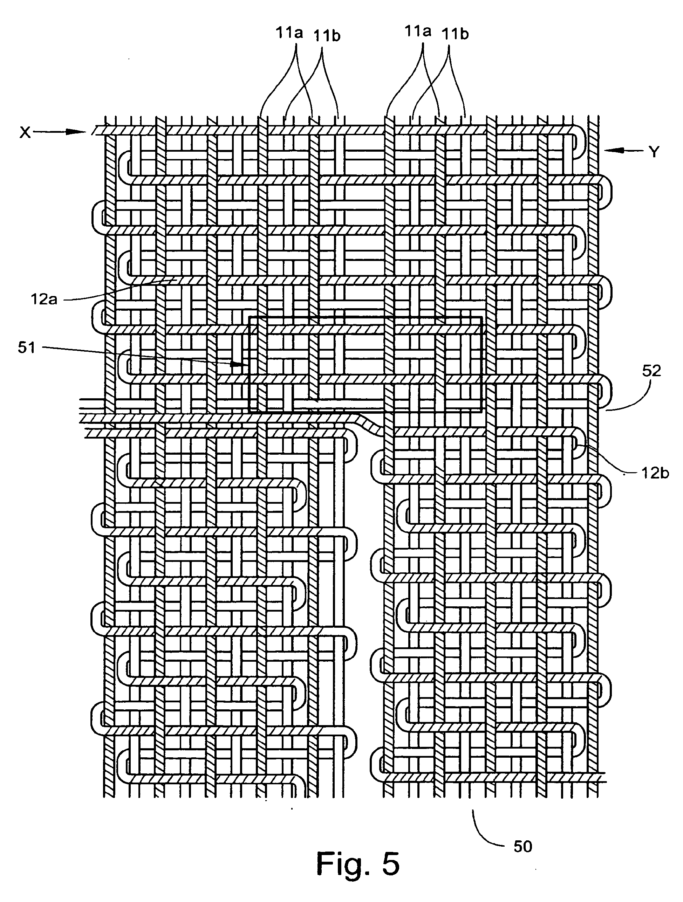

FIG. 5 is an open crotch weave pattern 50 that produces a tapered edge at a split useful in making an embodiment of a bifurcated tubular article. As shown in FIG. 5, the open crotch 51 at the bifurcation area 52 can be formed by gradually disengaging warp yarns 11a and 11b from fill yarns 12a. and 12b. The disengaging of the warp yarns 11a and 11b can be accomplished by dropping the desired warp yarns 11a and 11b from the end of the tubular flat-woven article such that the fill yarns 12a and 12b are not interwoven across the warp yarns 11a and 11b for that section of the pattern.

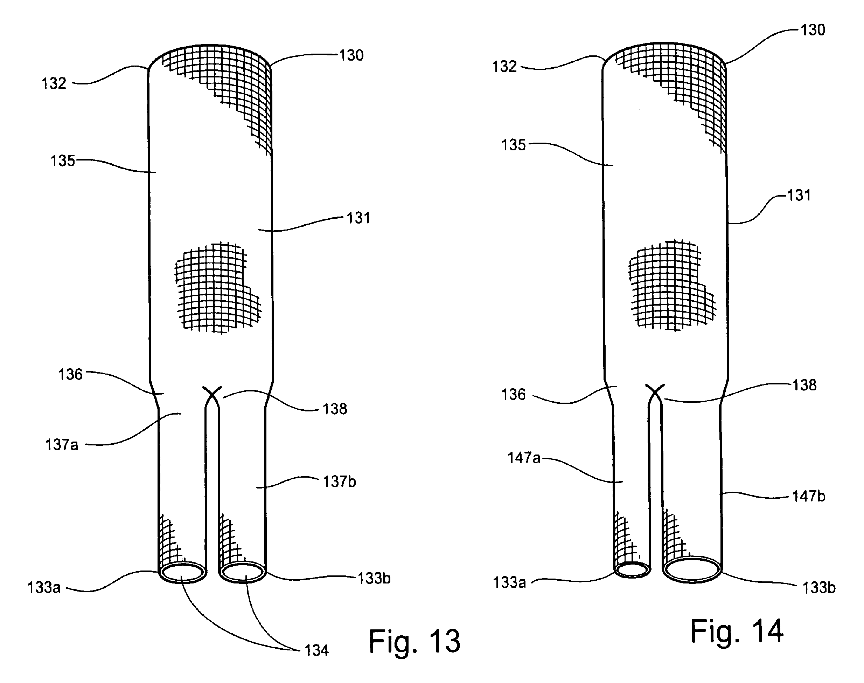

As shown in FIG. 5, the number of warp yarns at the crotch area can be split during the weaving process in order to split the tubular woven article from a primary tubular woven extent into a plurality of secondary woven extents. This splitting of warp yarns allows the transition at the crotch 138, as shown in FIG. 13, where the diameter of the tubular article transitions from a first inner diameter of the primary tubular woven extent 135, to two separate inner diameters representing the first and second secondary woven extents 137a and 137b.

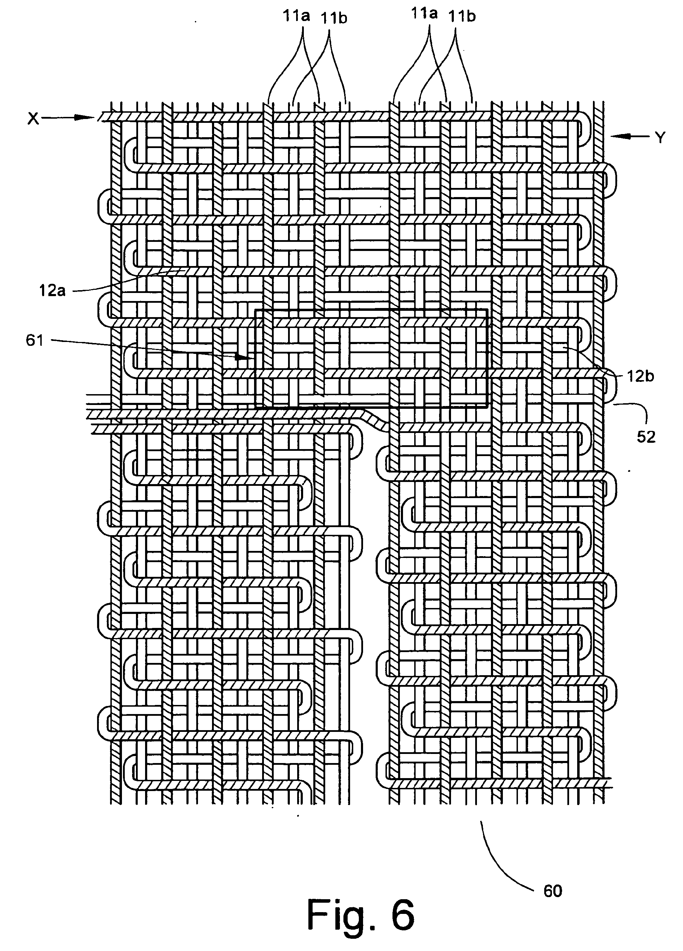

FIG. 6 shows a stitched crotch weave pattern 60 utilized in an embodiment of a method of the present invention to produce a stitched crotch 61 in a tubular article in an embodiment of the present invention. As shown in FIG. 6, the bifurcation area 52 can be formed by interweaving top layer warp yarns 11a and bottom layer warp yarns 11b together with fill yarns 12a and 12b to form a seam, or "selvage," comprising a single layer fabric at the bifurcation edges. A minimum of one warp yarn from each layer can be utilized. Additional warp yarns from either or both layers may be utilized to increase the width of the seam. The greater the number of warp yarns utilized, the greater the width, or size, of the seam. In some embodiments of the present invention, the size of the seam may be varied depending the intended end use of the tubular article.

In embodiments utilizing a stitched crotch weave pattern 60 as shown in FIG. 4, a seam can be made after weaving is complete and the tubular article is removed from the loom. The edges at a crotch or bifurcation may be sealed by sewing, welding, bonding, gluing, stapling, and/or other techniques suitable for sealing tubular articles.

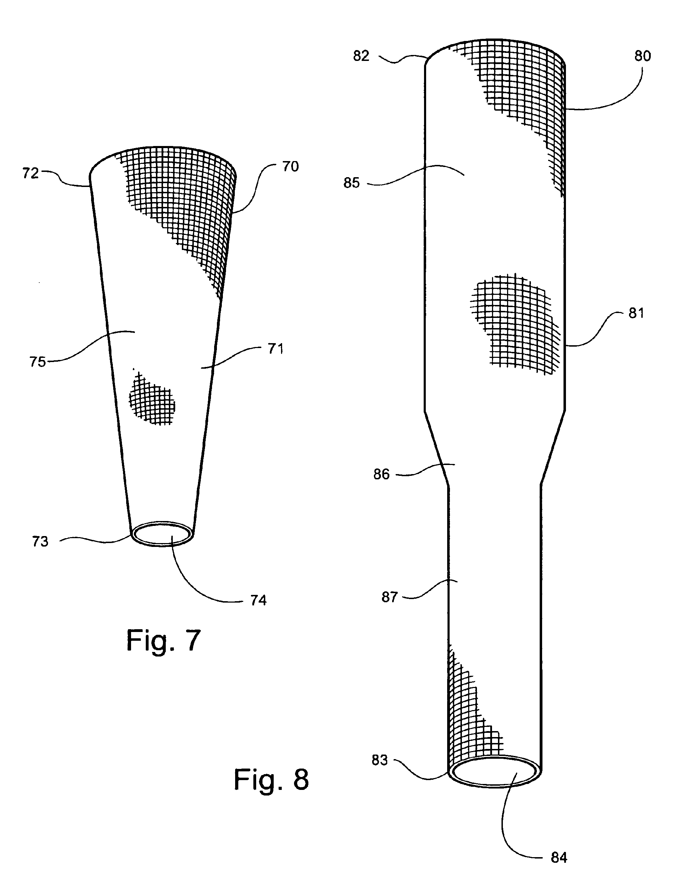

FIGS. 7-14 illustrate tubular articles having various shapes and configurations in embodiments of the present invention. The weaving pattern is not shown to scale. The tapered portions can comprise seams according to the present invention. Such embodiments may be used in bone filler material delivery devices.

Referring to FIG. 7, an embodiment of a tubular woven textile article 70 in accordance with the present invention is shown generally as a tapered article in a generally frustoconical shape. Tubular article 70 is a textile product formed of a woven synthetic fabric. Tubular article 70 is depicted in one embodiment in FIG. 7 which includes a generally tubular body 71 having a first end 72 and an opposed second end 73, defining therebetween an inner lumen 74 which permits passage of material through article 70. Tubular article 70 includes continuous transitional woven extent 75 extending between first end 72 and second end 73, and extending along the entire length of article 70. Tubular article 70 of FIG. 7 has a generally frustoconical shape, with first end 72 having a first tubular inner diameter and second end 73 having a second tubular inner diameter which is different than the inner diameter of first end 72. For example, first end 72 may have an inner diameter of 12 millimeters and second end 73 may have an inner diameter of 10 millimeters, with transitional woven portion 75 forming a gradual taper having successive changes in diameter throughout. As such, tubular article 70 gradually tapers from the 12 millimeter inner diameter of first end 72 to the 10 millimeter inner diameter of second end 73 along the length of transitional woven portion 75. The gradual tapering of transitional woven extent 75 can be accomplished by gradually disengaging and/or engaging a selected number of warp yarns from the weaving pattern during weaving of the article 70. Transitional woven extent 75 may include a seam along tapered edges to provide a substantially fluid-tight transition between first end 72 and second 73.

FIG. 8 shows a variation of the configuration of FIG. 7, with tubular article 80 in the form of a step-tapered tubular article having a tubular body 81 with a first end 82 and an opposed second end 83 defining an inner lumen 84 therebetween. In the embodiment of FIG. 8, tubular article 80 includes first woven extent 85 which defines a portion of tubular body 81 having a continuous first inner diameter and second woven extent 87 which defines a portion of tubular body 81 having a continuous second inner diameter which is different than the inner diameter of first woven extent 85. Tubular article 80 of FIG. 8 further includes transitional woven extent 86 adjacent and contiguous with first and second woven extents 85 and 87. In such an embodiment, tubular article 80 includes a constant diameter extending through first woven extent 85 and a constant diameter which is different than the inner diameter of first woven extent 85 which extends through second woven extent 87, and gradually tapers from the inner diameter of first woven extent 85 to the inner diameter of second woven extent 87 through the length of transitional woven extent 86. Transitional woven extent 86 may include a seam along tapered edges to provide a substantially fluid-tight transition between first woven extent 85 and second woven extent 87.

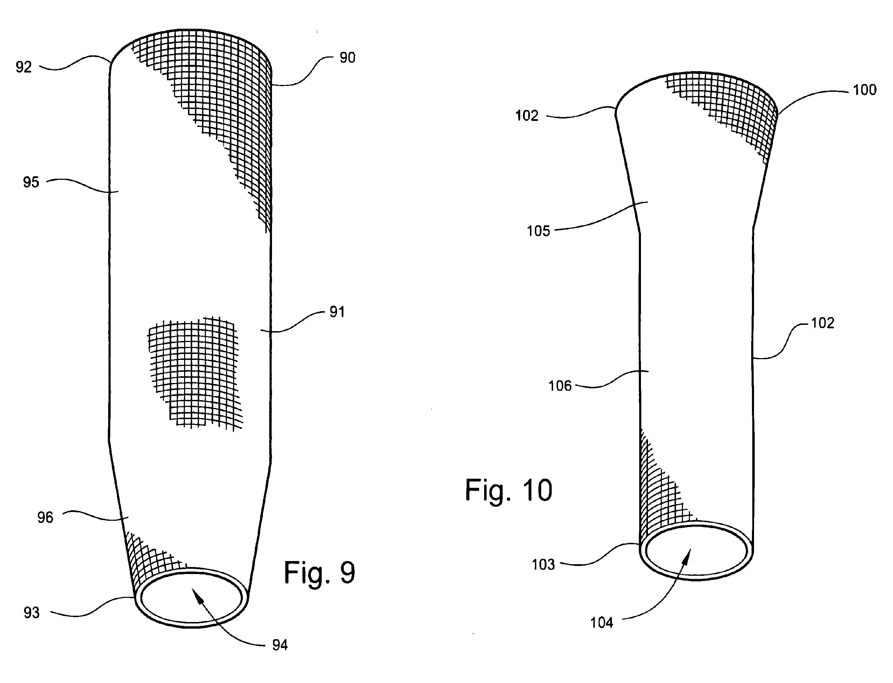

FIG. 9 shows another embodiment of the step-tapered configuration of FIG. 8, with tubular article 90 having a tubular body 91 with a first end 92 and an opposed second end 93 defining an inner lumen 94 therebetween. In the embodiment of FIG. 9, tubular article 90 includes a first woven extent 95 and a transitional woven extent 96, with the first woven extent 95 defining first end 92 and including a continuous inner diameter along the length thereof, and the transitional woven extent 96 defining second end 93 and including a gradual taper such that tubular article 90 gradually tapers from the inner diameter of first woven extent 95 to a second diameter at second end 93 which is different than the inner diameter of first woven extent 95. It is contemplated that such gradual tapering can be either an inward taper or an outward, or flared, taper. Transitional woven extent 96 may include a seam along tapered edges to provide a substantially fluid-tight transition between first woven extent 95 and second end 93.

FIG. 10 shows another embodiment of the configuration of tubular article 70 of FIG. 7, with tubular article 100 having a tubular body 101 with a first end 102 and an opposed second end 103 defining an inner lumen 104 therebetween. In the embodiment of FIG. 10, tubular article 100 includes a transitional woven extent 105 and a second woven extent 106, with the transitional woven extent 105 defining first end 102 and the second woven extent 106 including a continuous inner diameter along the length thereof, and defining second end 103. Further, transitional woven extent 105 includes a gradual taper such that tubular article 100 gradually tapers outwardly from the inner diameter of first end 102 to a second diameter at second end 103 which is different than the inner diameter of first end 102. Transitional woven extent 105 may include a seam along tapered edges to provide a substantially fluid-tight transition between first end 102 and second woven extent 106.

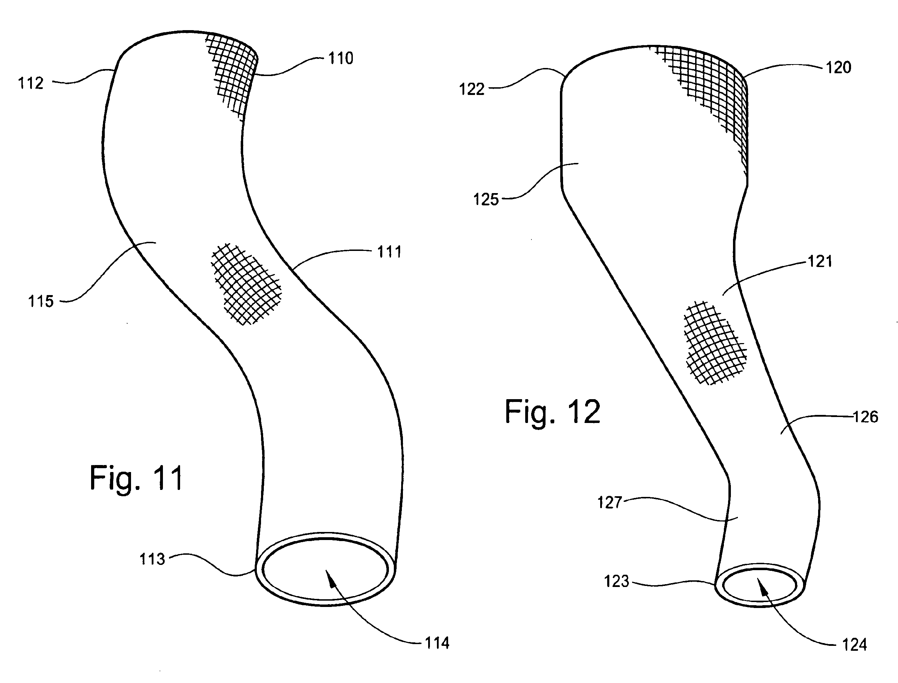

FIG. 11 depicts a sinusoidal shaped tubular article 110 having a tubular body 111 with a first end 112 and an opposed second end 113 defining an inner lumen 114 therebetween. In the embodiment of FIG. 11, tubular article 110 includes a continuous first woven extent 115, with the first woven extent 115 defining both first and second ends 112 and 113. First woven extent 115 has a continuous inner diameter along the length thereof, such that first end 112 and second end 113 have the same inner diameter. Tubular article 110 is shaped along its length in an "S" configuration, with tubular body 111 gradually changing direction as warp yarns on one edge of tubular article 110 during the weaving process are engaged or disengaged while the same portion of tubular body 111 on the other edge of tubular article 110 equally changes in the same direction as warp yarns are engaged or disengaged at this edge. Thus, as warp yarns at one edge of the tubular article 110 are disengaged as that edge and shape of the tubular article 110 gradually curve, the corresponding warp yarns at the opposite edge on the same pick are engaged. As the "S" shape again changes direction, the opposite may be true, that is, warp yarns at a given pick on one edge may be engaging as corresponding warp yarns at the other edge on the same pick may be disengaging. In order to maintain a constant diameter, the warp yarns at each of the edges of the tubular article change simultaneously by adding or engaging an equal number of warp yarns on one edge as the other edge loses or disengages warps. Thus, the total number of warp yarns within the tubular body wall remains constant during the weaving process. Continuous first woven extent 115 may include a seam along tapered edges to provide a substantially fluid-tight transition between first end 112 and second end 113.

FIG. 12 shows an embodiment of the present invention having a variation of the sinusoidal-shaped tubular article 110 shown in FIG. 11. Tubular article 120 in FIG. 12 includes a tubular body 121 with a first end 122 and an opposed second end 123 defining an inner lumen 124 therebetween. In the embodiment of FIG. 7, tubular article 120 includes first woven extent 125 having a first inner diameter and second woven extent 127 having a second inner diameter which is different than the inner diameter of first woven extent 125. Tubular article 120 further includes a transitional woven extent 126 adjacent first and second woven extents 125 and 127. For example, first woven extent 125 may include a woven tubular article section having an inner diameter of 12 millimeters and second woven extent 127 may include a woven tubular article section having an inner diameter of 10 millimeters, with transitional woven extent 126 forming a gradual taper. As such, tubular article 120 gradually tapers from the 12 millimeter inner diameter of first woven extent 125 to the 10 millimeter inner diameter of second woven extent 127 along the length of transitional woven extent 126. Tubular article 120 is shaped along its length in an "S" configuration similar to the manner in FIG. 11, with tubular body 121 gradually tapering in on one side of tubular article 120 during the weaving process, while the same portion of tubular body 121 on the other side of tubular article 120 tapers outwardly. Transitional woven extent 126 may include a seam along tapered edges to provide a substantially fluid-tight transition between first woven extent 125 and second woven extent 127.

FIGS. 13 and 14 illustrate embodiments of tubular articles of the present invention comprising bifurcations. As shown in the embodiments in FIGS. 13 and 14, a tubular woven bifurcated article 130 can include a generally tubular body 131 having a first end 132 and opposed second ends 133a and 133b, defining therebetween an inner lumen 134 which permits passage of material through the length of the tubular article 130. Bifurcated tubular article 130 includes a primary woven extent 135 having a first inner diameter, and further includes first and second secondary woven tubular extents 137a and 137b, each having an inner diameter which is different than the inner diameter of primary woven extent 135. The inner diameters of first and second secondary woven extents 137a and 137b can be the same as depicted in FIG. 13, or can be different as depicted in 147a and 147b of FIG. 14. Further, secondary woven extents 137a and 137b can be of the same general length as shown in FIGS. 13 and 14 or can be of different general lengths. Bifurcated tubular article 130 can further include bifurcated transitional woven extent 136 contiguous with primary woven extent 135 and first and second secondary woven extents 137a and 137b at crotch 138, forming a bifurcated arch. Bifurcated transitional woven extent 136 forms a gradual taper such that bifurcated tubular article 130 gradually tapers from the inner diameter of the primary woven extent 135 to the inner diameters of first and second secondary woven extents 137a and 137b along the length of bifurcated transitional woven extent 136. The gradual tapering of bifurcated transitional woven extent 136 can be accomplished by gradually disengaging and/or engaging a predetermined number of warp yarns from the weaving pattern during weaving of the tubular article, as discussed above. Bifurcated transitional woven extent 136 may include a seam along tapered edges to provide a substantially fluid-tight transition between the larger diameter, primary woven extent 135 and the first and second smaller diameter, secondary tubular extents 137a and 137b.

Further, during weaving of the bifurcated tubular article 130, two separate filling yarn shuttles (not shown) can be used to weave the two distinct secondary woven extents 137a and 137b. To form the gradual transition in the crotch 138, the shuttle designated for weaving of the secondary woven extent 137a selectively and gradually engages warp yarns designated for weaving of the secondary woven extent 137b. Likewise, the shuttle designated for weaving the secondary woven extent 137b selectively and gradually engages warp yarns designated for weaving of the secondary woven extent 137a. In this manner, the crotch 138 can be woven using a simultaneous tapering effect at the interface between the primary woven extent 135 and the secondary woven extents 137a and 137b. As such, a smooth contiguous surface transition can be obtained.

In vertebroplasty and/or kyphoplasty procedures, the surgeon may seek to treat a compression fracture of a vertebra by injecting bone cement such as polymethylmethacrylate (PMMA) into the fracture. In certain procedures, two PMMA precursor components (one powder and one liquid) can be mixed to produce a viscous bone cement prior to injection into a vertebral body. In such procedures, an embodiment of the bone filler material delivery device can include at least two secondary tubular extents 137a, 137b, as shown in FIG. 13, each of which can contain one of the PMMA precursor components. Each of the PMMA precursor components can be delivered from one of the secondary tubular extents 137a, 137b into a single primary tubular extent 131 where the precursors can be mixed and further delivered into the target vertebral body.

For example, such an embodiment of a bone filler device can include first and second secondary woven tubular extents 137a and 137b, to which is continuously woven a single primary woven tubular extent 131 having a first constant diameter. A transition tubular extent (not shown) can be continuously woven onto the first end 132 of the primary tubular extent 131, as shown in FIG. 13. A third secondary tubular woven extent (not shown) can be continuously woven onto the transition tubular extent. The diameter of the third secondary tubular woven extent can be smaller than the diameter of the primary tubular extent 131. The transition tubular extent can have a tapered edge along the transition extent formed by a weaving pattern in which a graduated number of warp yarns are disengaged as weaving proceeds. This weaving pattern can thus provide a graduated change in the number of warp yarns to form a graduated diameter along the transition extent. A seam can be woven along the tapered edge, such that the seam provides a substantially fluid-tight transition between the first tubular extent and the second tubular extent. In such a configuration, the bone filler device can contain separate precursors of PMMA in each of the first and second secondary woven tubular extents 137a and 137b, the precursors can be mixed in the primary woven tubular extent 131, and the bone cement mixture can be delivered through the smaller diameter third secondary tubular woven extent into a bone space in a minimally invasive procedure.

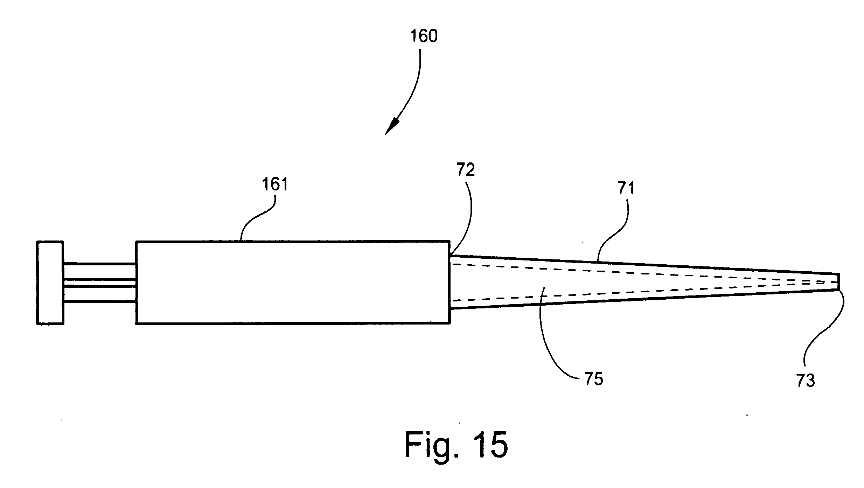

FIG. 15 is a view of an embodiment of a bone filler delivery device 160. A syringe 161 can be attached to the first end 72 of the tubular article 71. The tubular article 71 can include a continuous woven transitional tubular extent 75 extending along the entire length of article 71 and that tapers gradually from the first end 72 to the second end 73 of the tubular article 71. Tubular article 71 of FIG. 15 has a generally frustoconical shape similar to that in the embodiment in FIG. 7, with first end 72 having a first tubular inner diameter and second end 73 having a second tubular inner diameter which is different than the inner diameter of first end 72. The gradual tapering of transitional woven extent 75 can be accomplished by gradually disengaging and/or engaging a selected number of warp yarns from the weaving pattern during weaving of the article 71. Transitional woven extent 75 may include a seam along tapered edges to provide a substantially fluid-tight transition between first end 72 and second 73.

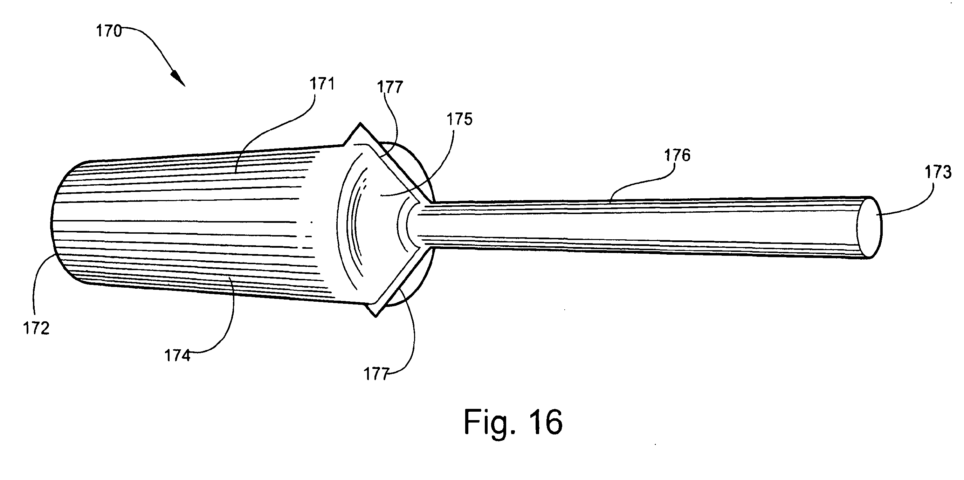

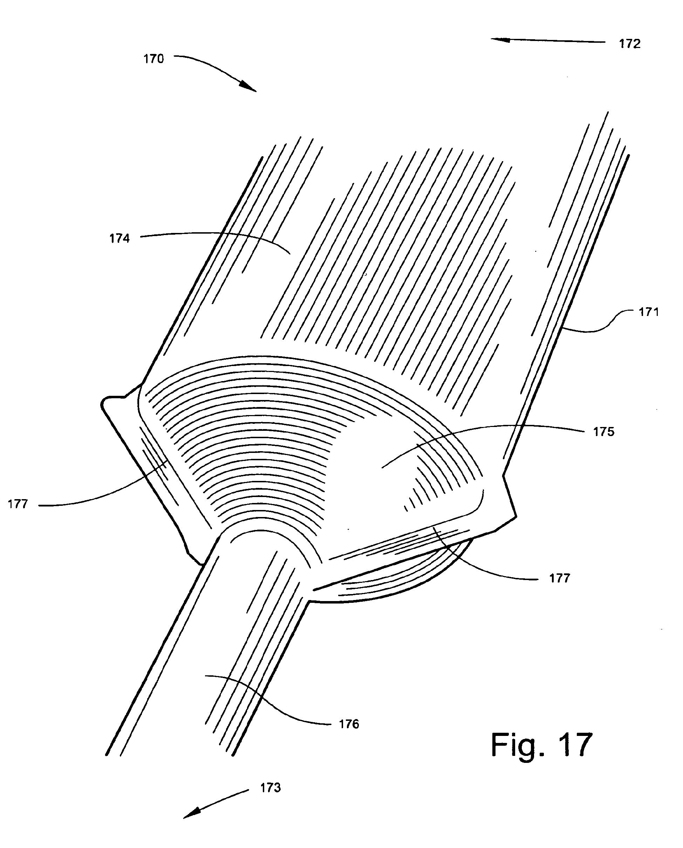

FIGS. 16 and 17 are views of another embodiment of a bone filler material delivery device 170 according to the present invention. As shown in this embodiment, the bone filler delivery device 170 can comprise a generally tubular body 171 of woven material extending from the first end 172 to the second end 173 of the device 170. The device 170 can include a first tubular extent 174 having a first substantially constant diameter and a tapered transition tubular extent 175 continuously woven from the first extent 174. A second tubular extent 176 having a second substantially constant diameter smaller than the first diameter can be continuously woven from the transition extent 175.

The transition tubular extent 175 can have a tapered edge along the transition extent 175 formed by a weaving pattern in which a graduated number of warp yarns can be disengaged as weaving proceeds. Certain warp yarns in the larger diameter first tubular extent 174 can be continuously woven through the transition tubular extent 175 into the smaller diameter second tubular extent 176. As some of the warp yarns in the first transition tubular extent 174 are "dropped" and not woven in the transition tubular extent 175, the tapered angle of the transition tubular extent 175 can be formed. As a result, such a weaving pattern can provide a graduated change in the number of warp yarns to form a graduated diameter along the transition extent 175.

In this manner, as the diameter of the transition tubular extent 175 gradually changes, the same density of warp yarns can be woven throughout the transition tubular extent 175 as in the first and second tubular extents 174, 176, respectively. Accordingly, the density of warp yarns can be uniform throughout the first, transition, and second tubular extents, 174, 175, 176, respectively. Thus, such embodiments of the tubular bone filler material delivery device 170 having tapered seam transitions can have more uniform characteristics such as porosity, strength, flexibility, and thickness along the entire length of the tubular body 171 as compared to conventional tubular articles.

A seam 177 can be woven along the tapered edge of the transition tubular extent 175, such that the seam 177 can provide a substantially fluid-tight transition between the first tubular extent 174 and the second tubular extent 176. As a result, the tapered transition areas, such as a seamed transition, can be essentially fluid-tight, or non-permeable, providing increased strength to the tubular body 171 and an improved barrier against leakage in tapered transition areas.

The diameter of the first tubular extent 174 and the diameter of the second tubular extent 176 can each be defined by a different number of warp yarns. That is, the first tubular extent 174 can comprise a first substantially constant diameter comprising a first number of warp yarns, and the second tubular extent 176 can comprise a second substantially constant diameter comprising a second number of warp yarns and having a diameter different than the first diameter. In some embodiments, the ratio of disengaged warp yarns to fill yarns can be high enough to allow the tapered edge and the transition tubular extent 176 to have a large angle of transition relative to the first and/or second tubular extents, 174, 176. For example, the ratio of disengaged warp yarns to fill yarns can allow the tapered edge to have an approximately 90 degree angle relative to the first and/or second tubular extents 174, 176. Thus, some embodiments of the present invention can provide a warp yarn to fill yarn reduction, or disengagement, ratio sufficiently high to allow highly angled transition areas.

In the embodiment shown in FIGS. 16 ad 17, the inwardly tapered transition tubular extent 175 comprises an angle of transition of about 45 degrees from the first tubular extent 174 to the second tubular extent 176. In other embodiments, the angle of inwardly tapered transition between the first and second tubular extents 174, 176, respectively, can be more or less than about 45 degrees, for example, between 0 and 90 degrees. The inward tapering of the woven transitional tubular extent 175 can be accomplished by gradually disengaging a selected number of warp yarns from the weaving pattern during weaving of the tubular article body 171. In other embodiments, the transition tubular extent 175 can flare outwardly at an angle of, for example, between 0 and 90 degrees. The transitional tubular extent 175 may include the seam 177 along tapered edges to provide a substantially fluid-tight transition between the first tubular extent 174 and the second tubular extent 176. In certain embodiments, the transitional tubular extent 175 may include a closure other than the continuously woven seam 177 along tapered edges to provide a substantially fluid-tight transition between the first tubular extent 174 and the second tubular extent 176.

The first, second, and transition tubular extents, 174, 175, 176, respectively, can have various dimensions. For example, in the embodiment shown in FIGS. 16 and 17, the first tubular extent 174 can have a diameter of about 16 mm and a length of about 3-4 inches (7.62 cm to 10.2cm). The second tubular extent 176 can have a diameter of about 3-4 mm and a length of about 2-3 inches (5.1cm to 7.62cm). The transition tubular extent 175 can have a diameter that varies gradually from the first tubular extent 174 to the second tubular extent 176 and a length of about one half to one inch (1.27 cm to 2.54 cm). The diameters and lengths of each of the tubular extents 174, 175, 176 can be larger or smaller and/or varied relative to each other than the dimensions shown in FIGS. 16 and 17. The warp yarn reduction ratio in the transition tubular extent may vary depending on the relative diameters of the first and second tubular extents 174, 176, respectively.

In the embodiment shown in FIGS. 16 and 17, the tubular body 171 of the bone filler material delivery device 170 can have a formed shape. For example, the device 170 can be removed in a flat woven condition from a loom, and the first and second tubular extents, 174, 176, respectively, heat set into the three dimensional tubular extents, 174, 176. In some embodiments, the seams 177 in the transition tubular extent 175 can be trimmed, for example, with scissors, to reduce the yarn ends extending beyond the seams 177. In addition, or alternatively, the seams 177 can be heated so as to fuse the yarn ends along the seams 177 together and provide a smooth seal about the seams 177.

The tubular bone filler delivery device 170 may be useful for delivering bone filler material, such as a bone cement, to a bony area in an internal region of a human or animal. Such an embodiment of the bone filler material delivery device 170 having a tapered transition 175 may be particularly useful for positioning the device through a surgical access path and delivering bone filler material to a treatment site in minimally invasive surgical procedures. For example, embodiments of the bone filler material delivery device 170 may be utilized, for example, in vertebroplasty and/or kyphoplasty procedures.

While a variety of shapes and configurations are shown in the drawings and described herein, any tubular, flat-woven article incorporating a gradually transitioning, continuously woven portion is contemplated by the present invention. The gradual tapering of the transitional woven portion or extent 175 can be accomplished in each of the embodiments by gradually disengaging and/or engaging a predetermined number of warp yarns 11 from the weaving pattern during weaving of the article as discussed above.

Any type of textile product can be used as the warp yarns 11 and fill yarns 12 of the present invention. Of particular usefulness in forming embodiments of tubular fabric medical devices of the present invention are synthetic materials such as thermoplastic polymers. Thermoplastic yarns suitable for use in the present invention include polyesters, polypropylenes, polyethylenes, polyurethanes, polytetrafluoroethylenes, as well as others. The yarns may be of the monofilament, multifilament, or spun type.

In some embodiments, the first and second tubular extents 174, 176, respectively, can be heat-set into formed, three-dimensional shapes. In some embodiments in which the yarns comprise thermoplastic yarns, yarn ends along the seam 177 can be heated and fused so as to provide a smooth seal about the seam 177.

Yarns utilized in fabric medical devices (such as the bone filler delivery device 170) of the present invention can comprise yarns known and generally utilized in the art for such devices. In general, the selection of yarn will depend on the intended end use application of the device. Yarns used in forming embodiments of fabric medical devices of the present invention may be flat, twisted or textured, and may have high, low or moderate shrinkage properties. Additionally, the yarn type and yarn denier can be selected to meet specific properties desired for the tubular article such as porosity, flexibility, and compliance. The yarn denier utilized in embodiments of fabric medical devices of the present invention can include a range of deniers from small to heavy.

In certain embodiments of the woven fabric medical device 170, the warp yarns 11 woven in the transition tubular extent 175 can be a high tenacity warp yarn 11. Tenacity can be defined as the tensile stress of a material based on the linear density of the unstrained material (ASTM). The breaking tenacity of a yarn or fabric is the tensile force at rupture per unit cross-sectional area or per unit linear density, for example, denier or tex. For example, a first yarn used in a woven medical device may have a tenacity, or tensile strength, of 3.8 gms / denier (or 3.8 gms / 0.11 tex). A second yarn having an equivalent size as the first yarn may have a tenacity, or tensile strength, of 7.0 gms / denier (or 7.0 gms / 0.11 tex). Thus, the second yarn can have a higher tenacity, or tensile strength, than the first yarn. As a result, the warp yarns 11 woven in the transition tubular extent 175 of the fabric medical device 170 can have a higher tenacity than the fill yarns 12 in the device 170. In this way, the increased tensile strength of the transition tubular extent 175 can enhance the stability of the transition tubular extent 175 as the device 170 is being inserted into, manipulated within, and/or withdrawn from a target treatment site in a person's body.

In certain embodiments, the tenacity of the higher tenacity warp yarns 11 in the transition tubular extent 175 can be at least 30% greater than the tenacity of the fill yarns 12 in the fabric medical device 170. In particular embodiments, the warp yarns 11 in the fabric medical device 170 can be polyester warp yarns 11. The polyester warp yarns 11 woven in the transition tubular extent 175 can have a higher tenacity than the fill yarns 12 in the device 170. In other embodiments, the warp and/or fill yarns can comprise yarns other than polyester yarns.

In certain embodiments of the woven fabric medical device 170, the fill yarns 12 (or weft yarns) woven in the transition tubular extent 175 can be a high tenacity fill yarn 12. That is, in certain embodiments, both the warp yarns 11 and the fill yarns 12 can be high tenacity yarns.

Some embodiments of the present invention can provide a method of making a woven fabric medical device (such as the bone filler delivery device 170). In an illustrative embodiment of such a method, a first tubular extent (174) having a first diameter can be woven using a first predetermined number of warp yarns 11. A transition tubular extent (175) can be continuously woven from the first tubular extent (174). The transition tubular extent (175) can be woven with a weaving pattern having a graduated change in a first predetermined number of warp yarns 11 to produce a tapered edge along the transition tubular extent (175). Weaving the graduated change in the number of warp yarns 11 can comprise disengaging predetermined warp yarns 11 from the weaving pattern. A second tubular extent (176) can then be continuously woven from the transition tubular extent (175) using a second predetermined number of warp yarns 11. A seam (177) can be created along the tapered edge such that the seam (177) provides a substantially fluid-tight transition between the first tubular extent (174) and the second tubular extent (176). In such an embodiment of a method, the warp yarns 11 can comprise top layer warp yarns 11a and bottom layer warp yarns 11b, and the seam (177) can further comprise the top layer warp yarns 11a woven together with the bottom layer warp yarns 11b along the tapered edge.

In another embodiment of a method of making a woven fabric medical device (170), a first tubular extent (174) having a first diameter can be woven using a first predetermined number of warp yarns 11. An open transition extent can be woven continuously from the first tubular extent (174). The woven fabric medical device (170) can include both warp yarns 11 and fill yarns 12. The fill yarns 12 can comprise top layer fill yarns 12a and bottom layer fill yarns 12b, and the fill yarns 12 at the tapered edge can be woven together onto the same layer, leaving the transition tubular extent open. A tapered, open transition extent can be woven by a weaving pattern having a graduated change in the first predetermined number of warp yarns 11. Such a graduated change in the number of warp yarns 11 can provide an unwoven tapered edge along the open transition extent. In some embodiments of such a method, the graduated change in the number of warp yarns 11 can be accomplished by disengaging a graduated number of warp yarns 11 from the weaving pattern. A second tubular extent (176) can be continuously woven from the open transition extent using a second predetermined number of warp yarns 11. Once weaving is completed, a seam (177) can be created along the tapered edge. The seam (177) can provide a substantially fluid-tight transition between the first tubular extent (174) and the second tubular extent (176). The open transition extent may be closed with a seam (177) utilizing a seam forming means after weaving is completed. For example, the seam forming means can comprise a seam (177) sewn along the tapered edge. In other embodiments, the seam forming means can comprise gluing, stapling, welding, and/or the like.

In another embodiment of a method of making a woven fabric medical device (170), a first tubular extent (174) having a first diameter can be woven using a first predetermined number of warp yarns 11. An open transition extent can be woven continuously from the first tubular extent (174). Such a tapered, open transition extent can be woven by a weaving pattern having a graduated change in the first predetermined number of warp yarns 11 to produce an open crotch. A plurality of secondary transition tubular extents (137a, 137b, for example) can be continuously woven at a transition with the first tubular extent (174). Once the woven fabric medical device (170) having an open crotch (138) in the transition tubular extent (175) is woven, a seam (177) can be created along the crotch (138). The seam (177) can provide the transition between the first tubular extent (174) and the plurality of secondary tubular extents (137a, 137b) with a substantially fluid-tight closure.

In some embodiments, the seam (177) can be created using a seam forming means to close the open crotch (138) after weaving is completed. For example, the seam (177) can be formed by sewing a seam (177) to close the open crotch (138).

Embodiments of methods for producing a woven fabric medical device (170) having seams (177) at tapered edges in transition areas can provide the advantage of allowing for an increased rate of transition. That is, certain embodiments of a woven fabric medical device (170) of the present invention may comprise more abruptly tapered portions than conventional tubular devices, rendering some embodiments of a woven fabric medical device (170) of the present invention more suitable for use in certain clinical applications.

Features of a fabric medical device having a tapered transition and/or method of making the device of the present invention may be accomplished singularly, or in combination, in one or more of the embodiments of the present invention. Although particular embodiments have been described, it should be recognized that these embodiments are merely illustrative of the principles of the present invention.

## Claims

1. A tubular fabric device (170), comprising:
a plurality of warp yarns (11) and fill yarns (12) woven together;
a first tubular extent (174) having a first diameter (172) and a formed shape ;
a second tubular extent (176) having a second (173), different diameter and a formed shape;
a transition tubular extent (175) having a graduated diameter between the first (174) and second tubular extents (176);
a tapered edge (21) along the transition tubular extent (175) formed by a weaving pattern in which a graduated number of warp yarns (11) are disengaged along the transition tubular extent (175);
a seam (177) woven along the tapered edge (21) and configured to provide a fluid-tight transition between the first tubular extent (174) and the second tubular extent (176);
**characterised in that** the warp yarns (11) in at least the transition tubular extent (175) have a tenacity higher than a tenacity of the fill yarns (12) and wherein the tubular fabric device is a medical device adapted for use in delivery of a fluid or semi-fluid material to a treatment site in a human or animal body.

2. The device (170) of claim 1, wherein the warp yarn tenacity is at least 30% higher than the fill yarn tenacity.

3. The device (170) of claim 1, wherein the plurality of warp yarns (11) comprises top layer warp yarns (11a) and bottom layer warp yarns (11b), and wherein the seam (177) comprises the top layer warp yarns (11a) and the bottom layer warp yarns (11b) woven together along the tapered edge (21).

4. The device (170) of claim 1, comprising a sufficiently high ratio of disengaged warp yarns (11) to fill yarns (12) such that the diameter of the transition tubular extent (175) graduates at an angle of at least 45 degrees between the first (174) and second tubular extents (176).

5. The device (170) of claim 1, wherein the first tubular extent (174) first diameter (172) and the second tubular extent (176) second diameter (173) each comprises a constant diameter.

6. The device (170) of claim 1, wherein the first tubular extent (174) first diameter (172) and the second tubular extent (176) second diameter (173) is each defined by a different number of warp yarns (11).

7. The device (170) of claim 1, comprising a flat-woven device.

8. The device (170) of claim 1, wherein the first (174) and second tubular extents (176), the transition tubular extent (175), and the seam (177) are woven together.

9. The device of claim 1, wherein the warp yarns (11) and fill yarns (12) comprise one or more thermoplastic polymers.

10. The device of claim 9, wherein the first (174) and second tubular extent (176) formed shapes comprise heat-set, three-dimensional shapes.

11. The device of claim 9, wherein the seam (177) comprises heated and fused yarn ends so as to provide a smooth seal about the seam (177).

12. The device (170) of claim 1, further comprising a frustoconical shape, wherein the first tubular extent (174) first diameter (172) is graduated, the second tubular extent (176) second diameter (173) is graduated, and the diameters of the first (172), second (173), and transition tubular extents (175) are each graduated in the same direction.

13. The device (170) of claim 1, the second tubular extent (176) comprising two secondary tubular extents (176) that bifurcate from the transition tubular extent (175), the tapered edge (21) forming a crotch in the transition tubular extent (175) between the two secondary tubular extents (176) by a weaving pattern in which a graduated number of warp yarns (11) are disengaged along the transition tubular extent (175).

14. The device of claim 1, comprising a bone filler delivery device adapted to deliver bone filler to a bony area in an internal body region.

15. A method for making a device (170) according to claims 1 to 14, comprising:
selecting a warp yarn (11) and a fill yarn (12) each having a tenacity;
weaving together a plurality of the warp yarns (11) and fill yarns (12) to form the device (170) having a first tubular extent (174) having a first diameter (172), a second tubular extent (176) having a second (173), different diameter, and a transition tubular extent (175) having a graduated diameter between the first (174) and second tubular extents (176);
disengaging a graduated number of warp yarns (11) along the transition tubular extent (175) to form a tapered edge (21);
weaving a seam (177) along the tapered edge (21) so as to provide a fluid-tight transition between the first tubular extent (174) and the second tubular extent (176), **characterised in that** the warp yarns (11) in at least the transition tubular extent (175) have a tenacity higher than a tenacity of the fill yarns (12).

16. The method of claim 15, wherein selecting a warp yarn (11) and a fill yarn (12) comprises selecting a warp yarn (11) and a fill yarn (12) comprising one or more thermoplastic polymers, the method comprising:
flat weaving the device (170) ; and
heat setting the first (174) and second tubular extents (176) into formed three-dimensional shapes.

## Patentansprüche

1. Rohrförmige Gewebevorrichtung (170) mit:
einer Vielzahl von Kettgarnen (11) und Füllgarnen (12), die miteinander verwebt sind;
einer ersten rohrförmigen Ausdehnung (174), die einen ersten Durchmesser (172) und eine ausgebildete Form aufweist;
einer zweiten rohrförmigen Ausdehnung (176), die einen zweiten, anderen Durchmesser (173) und eine ausgebildete Form aufweist;
einer rohrförmigen Übergangsausdehnung (175), die einen abgestuften Durchmesser zwischen der ersten (174) und zweiten rohrförmigen Ausdehnung (176) aufweist;
einer sich verjüngenden Kante (21), entlang der rohrförmigen Übergangsausdehnung (175), die durch ein Webmuster ausgebildet ist, in dem eine abgestufte Anzahl von Kettgarnen (11) entlang der rohrförmigen Übergangsausdehnung (175) lose sind;
einem Saum (177), der entlang der sich verjüngenden Kante (21) gewebt ist und eingerichtet ist, einen fluiddichten Übergang zwischen der ersten rohrförmigen Ausdehnung (174) und der zweiten rohrförmigen Ausdehnung (176) bereitzustellen;
**dadurch gekennzeichnet, dass** die Kettgarne (11) in zumindest der rohrförmigen Übergangsausdehnung (175) eine Zähigkeit aufweisen, die höher ist als eine Zähigkeit der Füllgarne (12) und wobei die rohrförmige Gewebevorrichtung eine medizinische Vorrichtung ist, die für eine Verwendung bei einer Zufuhr eines fluiden oder semifluiden Materials zu einer Behandlungsstelle in einem menschlichen Körper oder Tierkörper geeignet ist.

2. Vorrichtung (170) nach Anspruch 1, bei der die Kettgarnzähigkeit mindestens 30 % höher ist als die Füllgarnzähigkeit.

3. Vorrichtung (170) nach Anspruch 1, bei welcher die Vielzahl von Kettgarnen (11) eine obere Lage von Kettgarnen (11a) und eine untere Lage von Kettgarnen (11b) aufweist und bei welcher der Saum (177) die obere Lage von Kettgarnen (11a) und die untere Lage von Kettgarnen (11b) miteinander entlang der sich verjüngenden Kante (21) verwebt aufweist.

4. Vorrichtung (170) nach Anspruch 1, mit einem ausreichend hohen Verhältnis loser Kettgarne (11) zu Füllgarnen (12), sodass der Durchmesser der rohrförmigen Übergangsausdehnung (175) in einem Winkel von mindestens 45° zwischen der ersten (174) und zweiten rohrförmigen Ausdehnung (176) abgestuft ist.

5. Vorrichtung (170) nach Anspruch 1, bei welcher der erste Durchmesser (172) der ersten rohrförmigen Ausdehnung (174) und der zweite Durchmesser (173) der zweiten rohrförmigen Ausdehnung (176) jeweils einen konstanten Durchmesser aufweisen.

6. Vorrichtung (170) nach Anspruch 1, bei welcher der erste Durchmesser (172) der ersten rohrförmigen Ausdehnung (174) und der zweite Durchmesser (173) der zweiten rohrförmigen Ausdehnung (176) jeweils durch eine andere Anzahl von Kettgarnen (11) definiert ist.

7. Vorrichtung (170) nach Anspruch 1 mit einer flachgewebten Vorrichtung.

8. Vorrichtung (170) nach Anspruch 1, bei der die erste (174) und zweite rohrförmige Ausdehnung (176), die rohrförmige Übergangsausdehnung (175) und der Saum (177) miteinander verwebt sind.

9. Vorrichtung nach Anspruch 1, bei der die Kettgarne (11) und Füllgarne (12) einen oder mehrere thermoplastische Polymere aufweist.

10. Vorrichtung nach Anspruch 9, bei der die ausgebildeten Formen der ersten (174) und zweiten rohrförmigen Ausdehnung (176) wärmefestgelegte dreidimensionale Formen aufweisen.

11. Vorrichtung nach Anspruch 9, bei welcher der Saum (177) erwärmte und verschmolzene Garnenden aufweist, um eine glatte Versiegelung um den Saum (177) bereitzustellen.

12. Vorrichtung (170) nach Anspruch 1, ferner mit einer kegelstumpfartigen Form, wobei der erste Durchmesser (172) der ersten rohrförmigen Ausdehnung (174) abgestuft ist, der zweite Durchmesser (173) der zweiten rohrförmigen Ausdehnung (176) abgestuft ist und die Durchmesser der ersten Ausdehnung (172), zweiten Ausdehnung (173) und rohrförmigen Übergangsausdehnung (175) jeweils in der gleichen Richtung abgestuft sind.

13. Vorrichtung (170) nach Anspruch 1, bei der die zweite rohrförmige Ausdehnung (176) zwei sekundäre rohrförmige Ausdehnungen (176) aufweist, die sich von der rohrförmigen Übergangsausdehnung (175) aus aufgabeln, wobei die sich verjüngende Kante (21) einen Schritt in der rohrförmigen Übergangsausdehnung (175) zwischen den zwei sekundären rohrförmigen Ausdehnungen (176) durch ein Webmuster ausbildet, in dem eine abgestufte Anzahl von Kettgarnen (11) entlang der rohrförmigen Übergangsausdehnung (175) lose sind.

14. Vorrichtung nach Anspruch 1, mit einer Knochenersatzmaterialzuführvorrichtung, die angepasst ist, Knochenersatzmaterial zu einem Knochenbereich in einer inneren Körperregion zuzuführen.

15. Verfahren zum Herstellen einer Vorrichtung (170) nach einem der Ansprüche 1 bis 14, umfassend:
Auswählen eines Kettgarns (11) und eines Füllgarns (12), die jeweils eine Zähigkeit aufweisen;
Zusammenweben einer Vielzahl von Kettgarnen (11) und Füllgarnen (12), um die Vorrichtung (170) auszubilden, die eine erste rohrförmige Ausdehnung (174) mit einem ersten Durchmesser (172), eine zweite rohrförmige Ausdehnung (176) mit einem zweiten, anderen Durchmesser (173) und eine rohrförmige Übergangsausdehnung (175) mit einem abgestuften Durchmesser zwischen der ersten (174) und zweiten rohrförmigen Ausdehnung (176) aufweist;
Lösen einer abgestuften Anzahl von Kettgarnen (11) entlang der rohrförmigen Übergangsausdehnung (175), um eine sich verjüngende Kante (21) auszubilden;
Weben eines Saums (177) entlang der sich verjüngenden Kante (21), um einen fluiddichten Übergang zwischen der ersten rohrförmigen Ausdehnung (174) und der zweiten rohrförmigen Ausdehnung (176) bereitzustellen, **dadurch gekennzeichnet, dass** die Kettgarne (11) in zumindest der rohrförmigen Übergangsausdehnung (175) eine Zähigkeit aufweisen, die höher ist als eine Zähigkeit der Füllgarne (12).

16. Verfahren nach Anspruch 15, bei dem ein Auswählen eines Kettgarns (11) und eines Füllgarns (12) ein Auswählen eines Kettgarns (11) und eines Füllgarns (12) mit einem oder mehreren thermoplastischen Polymeren umfasst, wobei das Verfahren aufweist:
Flachweben der Vorrichtung (170); und
Wärmefestlegen der ersten (174) und zweiten rohrförmigen Ausdehnung (176) zu ausgebildeten dreidimensionalen Formen.

## Revendications

1. Dispositif en tissu tubulaire (170) comprenant :
une pluralité de fils de chaîne (11) et de fils de trame (12) tissés ensemble ;
une première étendue tubulaire (174) ayant un premier diamètre (172) et une forme formée ;
une seconde étendue tubulaire (176) ayant un second diamètre (173) différent et une forme formée ;
une étendue tubulaire de transition (175) ayant un diamètre graduellement variable entre les première (174) et seconde étendues tubulaires (176) ;
un bord progressivement rétréci (21) le long de l'étendue tubulaire de transition (175) formé par un motif de tissage dans lequel un certain nombre graduellement variable de fils de chaîne (11) sont laissés libres le long de l'étendue tubulaire de transition (175) ;
une couture (177) tissée le long du bord progressivement rétréci (21) et configurée pour fournir une transition étanche au fluide entre la première étendue tubulaire (174) et la seconde étendue tubulaire (176) ;
**caractérisé en ce que** les fils de chaîne (11) dans au moins l'étendue tubulaire de transition (175) ont une ténacité supérieure à une ténacité des fils de trame (12) et dans lequel le dispositif pour tissu tubulaire est un dispositif médical destiné à délivrer un matériau fluide ou semi-fluide à un site de traitement dans un corps humain ou animal.

2. Dispositif (170) selon la revendication 1, dans lequel la ténacité du fil de chaîne est au moins 30% supérieure à la ténacité au fil de trame.

3. Dispositif (170) selon la revendication 1, dans lequel la pluralité de fils de chaîne (11) comprend des fils de chaîne d'une couche supérieure (11a) et les fils de chaîne d'une couche inférieure (11b), et dans lequel la couture (177) comprend les fils de chaîne de la couche supérieure (11a) et les fils de chaîne de la couche inférieure (11b) tissés ensemble le long du bord progressivement rétréci (21).

4. Dispositif (170) selon la revendication 1, comprenant un rapport des fils de chaîne (11) laissés libres sur les fils de trame (12) suffisamment élevé de sorte que le diamètre de l'étendue tubulaire de transition (175) est graduellement variable à un angle d'au moins 45 degrés entre les première (174) et seconde étendues tubulaires (176).

5. Dispositif (170) selon la revendication 1, dans lequel le premier diamètre (172) de la première étendue tubulaire (174) et le second diamètre (173) de la seconde étendue tubulaire (176) comprennent chacun un diamètre constant.

6. Dispositif (170) selon la revendication 1, dans lequel le premier diamètre (172) de la première étendue tubulaire (174) et le second diamètre (173) de la seconde étendue tubulaire (176) sont chacun définis par un nombre différents de fils de chaîne (11).

7. Dispositif (170) selon la revendication 1, comprenant un dispositif tissé à plat.

8. Dispositif (170) selon la revendication 1, dans lequel les première (174) et seconde étendues tubulaires (176), l'étendue tubulaire de transition (175) et la couture (177) sont tissées ensemble.

9. Dispositif selon la revendication 1, dans lequel les fils de chaîne (11) et les fils de trame (12) comprennent un ou plusieurs polymères thermoplastiques.

10. Dispositif selon la revendication 9, dans lequel les formes formées des première (174) et seconde étendues tubulaires (176) comprennent des formes tridimensionnelles thermofixées.

11. Dispositif selon la revendication 9, dans lequel la couture (177) comprend des extrémités de fil chauffées et fusionnées afin de fournir un joint régulier autour de la couture (177).

12. Dispositif (170) selon la revendication 1, comprenant en outre une forme tronconique, dans lequel le premier diamètre (172) de la première étendue tubulaire (174) est graduellement variable, le second diamètre (173) de la seconde étendue tubulaire (176) est graduellement variable, et les diamètres des première (172) et seconde (173) étendues tubulaires ainsi que des étendues tubulaires de transition (175) sont chacun graduellement variables dans la même direction.

13. Dispositif (170) selon la revendication 1, la seconde étendue tubulaire (176) comprenant deux étendues tubulaires secondaires (176) qui bifurquent à partir de l'étendue tubulaire de transition (175), le bord progressivement rétréci (21) formant une fourche dans l'étendue tubulaire de transition (175) entre les deux étendues tubulaires secondaires (176) par un motif de tissage dans lequel un nombre graduellement variable de fils de chaîne (11) sont laissés libres le long de l'étendue tubulaire de transition (175).

14. Dispositif selon la revendication 1, comprenant un dispositif de délivrance de matière de remplissage osseuse adapté pour délivrer la matière de remplissage osseuse à une zone osseuse dans une région corporelle interne.

15. Procédé pour fabriquer un dispositif (170) selon les revendications 1 à 14, comprenant les étapes consistant à :
sélectionner un fil de chaîne (11) et un fil de trame (12) ayant chacun une ténacité ;
tisser conjointement une pluralité de fils de chaîne (11) et de fils de trame (12) afin de former le dispositif (170) ayant une première étendue tubulaire (174) ayant un premier diamètre (172), une seconde étendue tubulaire (176) ayant un second diamètre (173) différent et une étendue tubulaire de transition (175) ayant un diamètre graduellement variable entre les première (174) et seconde étendues tubulaires (176) ;
laisser libres un nombre graduellement variable de fils de chaîne (11) le long de l'étendue tubulaire de transition (175) pour former un bord progressivement rétréci (21) ;
tisser une couture (177) le long du bord progressivement rétréci (21) afin de fournir une transition étanche au fluide entre la première étendue tubulaire (174) et la seconde étendue tubulaire (176), **caractérisé en ce que** les fils de chaîne (11) dans au moins l'étendue tubulaire de transition (175) ont une ténacité supérieure à une ténacité des fils de trame (12).

16. Procédé selon la revendication 15, dans lequel l'étape consistant à sélectionner un fil de chaîne (11) et un fil de trame (12) comprend l'étape consistant à sélectionner un fil de chaîne (11) et un fil de trame (12) comprenant un ou plusieurs polymères thermoplastiques, le procédé comprenant les étapes consistant à :
tisser le dispositif (170) à plat ; et
thermofixer les première (174) et seconde étendues tubulaires (176) en formes tridimensionnelles formées.
